# EUROPEAN PATENT APPLICATION

(11) **EP 0 528 486 A2**
(43) Date of publication of application: **24.02.1993**
(21) Application number: 92202446.8
(22) Date of filing: 08.08.1992
(51) Int. Cl.: C07K 5/10, A61K 37/64

(54) **Non-substrate inhibitors of farnesyl protein transferase**

(30) Priority: 16.08.1991 US 746389
(71) Applicant: MERCK & CO. INC., Rahway New Jersey 07065-0900 (US)
(72) Inventor: Gibbs, Jackson B., Chalfont, PA 18914 (US); Rands, Elaine, Ambler, PA 19002 (US); Pompliano, David L., Lansdale, PA 19446 (US); Garsky, Victor M., Blue Bell, PA 19422 (US)
(74) Representative: Barrett-Major, Julie Diane

(57) **Abstract**

The present invention is directed to compounds which inhibit farnesyl-protein transferase (FTase) and the farnesylation of the oncogene protein Ras. The invention is further directed to chemotherapeutic compositions containing the compounds of this invention and methods for inhibiting farnesyl-protein transferase and the farnesylation of the oncogene protein Ras.

## Description

### BACKGROUND OF THE INVENTION

The Ras gene is found activated in many human cancers, including colorectal carcinoma, exocrine pancreatic carcinoma, and myeloid leukemias. Biological and biochemical studies of Ras action indicate that Ras functions like a G-regulatory protein, since Ras must be localized in the plasma membrane and must bind with GTP in order to transform cells (Gibbs, J. et al., Microbiol. Rev. 53:171-286 (1989). Forms of Ras in cancer cells have mutations that distinguish the protein from Ras in normal cells.

At least 3 post-translational modifications are involved with Ras membrane localization, and all 3 modifications occur at the C-terminus of Ras. The Ras C-terminus contains a sequence motif termed a "CAAX" or "Cys-Aaa¹-Aaa²-Xaa" box (Aaa is an aliphatic amino acid, the Xaa is any amino acid) (Willumsen et al., Nature 310:583-586 (1984)). Other proteins having this motif include the Ras-related GTP-binding proteins such as Rho, fungal mating factors, the nuclear lamins, and the gamma subunit of transducin.

Farnesylation of Ras by the isoprenoid farnesyl diphosphate (FPP) occurs in vivo on Cys to form a thioether linkage (Hancock et al., Cell 57:1167 (1989); Casey et al., Proc. Natl. Acad. Sci. USA 86:8323 (1989)). In addition, Ha-Ras and N-Ras are palmitoylated via formation of a thioester on a Cys residue near a C-terminal Cys farnesyl acceptor (Gutierrez et al., EMBO J. 8:1093-1098 (1989); Hancock et al., Cell 57: 1167-1177 (1989)). Ki-Ras lacks the palmitate acceptor Cys. The last 3 amino acids at the Ras C-terminal end are removed proteolytically, and methyl esterification occurs at the new C-terminus (Hancock et al., ibid). Fungal mating factor and mammalian nuclear lamins undergo identical modification steps (Anderegg et al., J.Biol. Chem. 263:18236 (1988); Farnsworth et al., J. Biol. Chem. 264:20422 (1989)).

Inhibition of Ras farnesylation in vivo has been demonstrated with lovastatin (Merck & Co., Rahway, NJ) and compactin (Hancock et al., ibid; Casey et al., ibid; Schafer et al., Science 245:379 (1989)). These drugs inhibit HMG-CoA reductase, the rate limiting enzyme for the production of polyisoprenoids and the farnesyl diphosphate precursor. It has been shown that a farnesyl-protein transferase using farnesyl diphosphate as a precursor is responsible for Ras farnesylation. (Reiss et al., Cell, 62: 81-88 (1990); Schaber et al., J. Biol. Chem., 265:14701-14704 (1990); Schafer et al., Science, 249: 1133-1139 (1990); Manne et al., Proc. Natl. Acad. Sci USA, 87: 7541-7545 (1990)).

Inhibition of farnesyl-protein transferase and, thereby, of farnesylation of the Ras protein, blocks the ability of Ras to transform normal cells to cancer cells. The compounds of the invention inhibit Ras localization in the plasma membrane and, thereby, generate soluble Ras which, as indicated infra, can act as a dominant negative inhibitor of Ras function. While soluble Ras in cancer cells can become a dominant negative inhibitor, soluble Ras in normal cells would not be an inhibitor.

A cytosol-localized (no Cys-Aaa¹-Aaa²-Xaa box membrane domain present) and activated (impaired GTPase activity, staying bound to GTP) form of Ras acts as a dominant negative Ras inhibitor of membrane-bound Ras function (Gibbs et al., Proc. Natl. Acad. Sci. USA 86:6630-6634(1989)). Cytosol-localized forms of Ras with normal GTPase activity do not act as inhibitors. Gibbs et al., ibid, showed this effect in Xenopus oocytes and in mammalian cells.

Administration of compounds of the invention to block Ras farnesylation not only decreases the amount of Ras in the membrane but also generates a cytosolic pool of Ras. In tumor cells having activated Ras, the cytosolic pool acts as another antagonist of membrane-bound Ras function. In normal cells having normal Ras, the cytosolic pool of Ras does not act as an antagonist. In the absence of complete inhibition of farnesylation, other farnesylated proteins are able to continue with their functions.

Farnesyl-protein transferase activity may be reduced or completely inhibited by adjusting the compound dose. Reduction of farnesyl-protein transferase enzyme activity by adjusting the compound dose would be useful for avoiding possible undesirable side effects such as interference with other metabolic processes which utilize the enzyme.

These compounds and their analogs are inhibitors of farnesyl-protein transferase. Farnesyl-protein transferase utilizes farnesyl diphosphate to covalently modify the Cys thiol group of the Ras CAAX box with a farnesyl group. Inhibition of farnesyl diphosphate biosynthesis by inhibition of HMG-CoA reductase blocks Ras membrane localization in vivo and inhibits Ras function. Inhibition of farnesyl-protein transferase is more specific and is attended by fewer side effects than is the case for a general inhibitor of isoprene biosynthesis.

Previously, it has been demonstrated that tetrapeptide mimics of the CAAX box inhibit Ras farnesylation (Schaber et al., ibid; Reiss et. al., ibid; Reiss et al., PNAS, 88:732-736 (1991)). The peptides appear to act as substrates. Since farnesylated tetrapeptides lose potency by about 15-fold (Schaber, et al., ibid), it is desirable to develop tetrapeptides which act as non-substrate compounds and therefore act as true inhibitors. The novel feature of the present invention is the discovery of a class of tetrapeptides that are not substrates or are poor substrates and therefore act as non-substrate inhibitors.

It is, therefore, an object of this invention to develop compounds which will inhibit farnesyl-protein transferase and the farnesylation of the oncogene protein Ras. It is a further object of this invention to develop chemotherapeutic compositions containing the compounds of this invention, and methods for producing the compounds of this invention.

### SUMMARY OF THE INVENTION

The present invention includes compounds which inhibit farnesyl-protein transferase and the farnesylation of the oncogene protein Ras, by serving as non-substrate inhibitors, chemotherapeutic compositions containing the compounds of this invention, and methods for producing the compounds of this invention.

The compounds of this invention are illustrated by the formula:

Cys - Xaa¹ - Xaa² - Xaa³ - NRR¹

wherein:
Cys is the amino acid Cysteine;
Xaa¹ is any amino acid in the natural L-isomer form;
Xaa² is any amino acid in the natural L-isomer form; and
Xaa³-NRR¹ is an amide of any amino acid in the natural L-isomer form, wherein R and R¹ are independently selected from hydrogen, C₁-C₁₂ alkyl, aralkyl, or unsubstituted or substituted aryl; or the pharmaceutically acceptable salts thereof.

### DETAILED DESCRIPTION OF THE INVENTION

The compounds of this invention are useful in the inhibition of farnesyl-protein transferase and the farnesylation of the oncogene protein Ras by serving as non-substrate inhibitors. The compounds of this invention are illustrated by the formula:

Cys - Xaa¹ - Xaa² - Xaa³ - NRR¹

wherein:
Cys is the amino acid Cysteine;
Xaa¹ is any amino acid in the natural L-isomer form;
Xaa² is any amino acid in the natural L-isomer form; and
Xaa³-NRR¹ is an amide of any amino acid in the natural L-isomer form, wherein R and R¹ are independently selected from hydrogen, C₁-C₁₂ alkyl, aralkyl, or unsubstituted or substituted aryl;
or the pharmaceutically acceptable salts thereof.

The preferred compounds of this invention include primary amides such as CVLS-NH₂ (SEQ ID NO: 1) and CVFM-NH₂ (SEQ ID NO: 2).

In the present invention, the amino acids are identified both by conventional 3 letter and single letter abbreviations as indicated below:

| | | |
|---|---|---|
| Alanine | Ala | A |
| Arginine | Arg | R |
| Asparagine | Asn | N |
| Aspartic acid | Asp | D |
| Asparagine or aspartic acid | Asx | B |
| Cysteine | Cys | C |
| Glutamine | Gln | Q |
| Glutamic acid | Glu | E |
| Glutamine or glutamic acid | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The pharmaceutically acceptable salts of the compounds of this invention include the conventional non-toxic salts of the compounds of this invention as formed, e.g., from non-toxic inorganic or organic acids. For example, such conventionl non-toxic salts include those derived from inorganic acids such as hydrochloric, hydrobromic, sulfuric, sulfamic, phosphoric, nitric and the like: and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, and the like.

The pharmaceutically acceptable salts of the present invention can be synthesized from the compounds of this invention which contain a basic moiety by conventional chemical methods. Generally, the salts are prepared by reacting the free base with stoichiometric amounts or with an excess of the desired salt-forming inorganic or organic acid in a suitable solvent or various combinations of solvents.

Polyisoprenylation of Ras- [³H]Farnesyl diphosphate (FPP) (20 Ci/mmol) was purchased from New England Nuclear. Farnesyl-protein transferase activity assays were carried out at 30°C unless noted otherwise. A typical reaction contained (in a final volume of 50 µl):[³H]farnesyl diphosphate, Ras protein (3.5µM), 50 mM HEPES, pH 7.5, 5 mM MgCl₂ 5 mM dithiothreitol and farnesyl-protein transferase. After thermally pre-equilibrating the assay mixture in the absence of enzyme, reactions were initiated by the addition of farnesyl-protein transferase and stopped at timed intervals by the addition of 1M HCl in ethanol (1 mL). The quenched reactions were allowed to stand for 15 minutes (to complete the precipitation process). After adding 2 mL of 100% ethanol, the reactions were vacuum-filtered through Whatmann GF/C filters. Filters were washed four time with 2 mL aliquots of 100% ethanol, mixed with scintillation fluid (10 mL) and then counted in a Beckmann LS3801 scintillation couter.

For inhibition studies, assays were run as described above, except putative inhibitors were added at the concentration indicated, IC₅₀ values were determined with both farnesyl-protein transferase substrates at their K_{M} concentrations. The K_{M} for Ras protein substrate having the CAAX sequence, CVLS, is 3.5 µM and the K_{M} for FPP is 0.25µM.

When a peptide was used as the acceptor substrate, there were two changes to the assay: (1) Ras protein was excluded and, (2) the reactions were stopped by the additon of one tenth volume of 0.5 M EDTA. A portion of the reaction mixture was spotted onto a TLC plate (silica gel 60 thin layer chromatography plates (20 cm x 20 cm, 0.25 mm layer thickness) were from E. Merck (Darrnstadt) and eluted with either ethyl acetate:pyridine: acetic acid:water (10:5:1:3) or n-propanol:water (7:3) elution systems. After drying under vacuum, the plates were sprayed with En³Hance and exposed to X-ray film at -70°C.

Ras proteins used as substrates were expressed in E. coli and purified. (Gibbs et al. Proc. Natl. Acad. Sci. USA 85:5026-5030(1988). Farnesyl-protein transferase was prepared from bovine brain. All purification steps were performed at 4°C. Cerebral lobes from bovine brains were homogenized in lysis buffer containing 50 mM Tris-Cl, pH 8.0, 1 mM EGTA, 1 mM MgCl₂, 5 mM dithiothreitol, 10 µg/mL aprotinin, 0.5 mM phenylmethyl sulfonyl flouride (PMSF), 2 µg/mL antipain and 2 µg/ml leupeptin. Cellular debris and membranes were removed by centrifugation (10,000g for 20 min followed by 100,000g for 30 minutes). The supernatant was loaded directly onto a column (30 cm x 20 cm²) of DEAE-Sephacel that had been equilibrated with lysis buffer. The column was washed with the same buffer and proteins were eluted with a linear gradient of NaCl (0-500 mM, 1 L + 1L) in the same buffer. Fractions (20 mL) were collected and those containing farnesyl-protein transferase activity were pooled. Each pool was then applied to an ω-Aminooctyl agarose column (30 cm x 4.9 cm²) and eluted with a linear gradient of NaCl (0-500 mM, 500 mL + 500 mL) in lysis buffer. Fractions containing farnesyl-protein transferase were pooled. For most assays, further partial purification was achieved by HPLC using a Mono Q HR 10/10 column and eluting farnesyl-protein transferase activity with a 0-0.3M NaCl gradient over 85 min. at 2 ml/min.

To prepare pure enzyme, the nonapeptide, KSLTGCVIM, was covalently bound to Affigel-10 under anhydrous conditions as described by the manufacturer (15 µmol peptide per 1 mL of Affigel-10). Unreacted resin was treated with ethanolamine. After multiple passes of ω-aminooctyl-agarose-column-purified protein over the peptide column, the column was washed and eluted as described by Reiss, et al., (1990) ibid, to recover farnesyl-protein transferase activity.

The compounds of the invention can be synthesized from their constituent amino acids by conventional peptide synthesis techniques, preferably by solid-phase technology. The peptides are then purified by reverse-phase high performance liquid chromatography (HPLC).

Standard methods of peptide synthesis are disclosed, for example, in the following works: Schroeder et al., "The Peptides", Vol. I, Academic Press 1965, or Bodanszky et al., "Peptide Synthesis", Interscience Publishers, 1966, or McOmie (ed.) "Protective Groups in Organic Chemistry", Plenum Press, 1973, or Barany et al., "The Peptides: Analysis, Synthesis, Biology" 2, Chapter 1, Academic Press, 1980, or Stewart et al., "Solid Phase Peptide Synthesis", Second Edition, Pierce Chemical Company, 1984. The teachings of these works are hereby incorporated by reference.

Peptides were prepared with an Applied Biosystems model 430A peptide synthesizer, converted to the corresponding amides by standard procedures, purified by reverse-phase HPLC, and characterized by amino acid analysis and fast atom bombardment mass spectrometry. Chemical farnesylation was achieved by reacting trans, trans-farnesyl bromide (Aldrich) with unprotected peptide.

Farnesylation of Ras in vitro- To evaluate in vitro polyisoprenylation of Ras, two engineered S. cerevisiae RAS gene products were used, [Leu⁶⁸]-RAS1(term.) and [Leu⁶⁸]RAS1(term.)CVLS (CVLS=Cys-Val-Leu-Ser) described by Gibbs et al., Proc. Natl. Acad. Sci. USA 86:6630-6634 (1989). Both proteins contain the first 184 amino acids of yeast RAS1 which does not have any Cys residues. [Leu⁶⁸]RAS1(term.) ends with a Pro substitution for Leu-185.
[Leu⁶⁸]RAS1
(term.)CVLS has the final Pro residue replaced by an additional seven C-terminal residues having the sequence -Ser-Leu-Lys-Cys-Val-Leu-Ser wherein this protein is herein called Ras-CVLS. Purified Ras protein (3.5 µM) was incubated with partially pure enzyme in the presence of 0.25 µM [³H]FPP. Radioactive precursor was incorporated into Ras-CVLS. No detectable labeling was observed in reactions lacking Ras or having [Leu⁶⁸]RAS1(term.) suggesting that modification was occurring at the unique C-terminal region of Ras-CVLS. Ras-CVLS was positively identified as the acceptor protein by immunoprecipitation with the Ras-specific monoclonal antibody Y13-259. Both normal and oncogenic mammalian Ha Ras proteins also served as substrates.

CAAX tetrapeptides and other tetrapeptides including Cys-Xaa¹-Xaa²-Xaa³-NRR¹ were tested as farnesylation substrates using [³H]FPP and partially pure bovine brain farnesyl-protein transferase. Our earlier findings with the hepta-peptide SSGCVLS suggested that it served as a substrate for isoprenylation (Schaber et al., ibid). Thin layer chromatography (TLC) conditions were developed which resolved the substrates farnesyl diphosphate (R_{f} = 0.06) and CVLS (R_{f} = 0.28) and product S-farnesyl-CVLS (R_{f} = 0.58). These R_{f} values reflect TLC conditions using ethylacetate:pyridine: acetic acid:water (10:5:1:3) as the eluent. In another elution system, n-propanol:water (7:3), farnesyl-diphosphate (R_{f}= 0.18) was readily seperated from farnesylated tetrapeptides (examples: CAIS (R_{f}=0.84) and CAIM (R_{f}=0.83)). Quenched reaction mixtures containing peptide, farnesyl-diphosphate and farnesyl-protein transferase were separated by TLC followed by visualization of the radioactive species by autoradiography. In the control reaction (no peptide) only unreacted starting material and farnesyl-diphosphate could be seen, indicating that the partially purified enzyme preparation had little phosphatase activity. In the presence of peptide CVLS, a radioactive spot was observed that co-migrated with the S-farnesyl-CVLS standard. Control peptides having a Cys to Ser substitution were not substrates.

The results of these experiments are shown in Table 1. The criticality of Cys-Xaa¹-Xaa²-Xaa³-NRR¹ as defined in this specification is shown.

**Table 1**

| Peptide (200µM) | Farnesylation Substrate |
|---|---|
| CVLS | + |
| SVLS | - |
| CVLS-NH₂ (SEQ ID NO: 1) | - |
| CVFM-NH₂ (SEQ ID NO: 2) | - |
| + > 90% [³H] farnesyl diphosphate converted to [³H] farnesyl tetrapeptide - < 1% conversion | |

The indicated peptides at 200 µM were incubated with partially pure bovine farnesyl-protein transferase and 0.5 µM [3H] FPP for 30 min. at 30°C. Autoradiographic exposure time was overnight (15-20 hr).

In order to determine whether tetrapeptides that were not substrates could bind to farnesyl-protein transferase, tetrapeptides were tested for the ability to inhibit Ras farnesylation in vitro (Table 2).

**Table 2**

| Inhibition of Ras Farnesylation by Tetrapeptides | |
|---|---|
| Compound | IC₅₀ (µM) |
| CVLS | 2 |
| CVLS-NH₂ (SEQ ID NO: 1) | 16 |
| CVFM-NH₂ (SEQ ID NO: 2) | 2.3 |

Farnesyl-protein transferase from bovine brain was chromatographed on DEAE-Sephacel (Pharmacia, 0-0.8 M NaCl gradient elution), N-octyl agarose (Sigma, 0-0.6 M NaCl gradient elution), and a mono Q HPLC column (Pharmacia, 0-0.3 M NaCl gradient). Ras-CVLS at 3.5 µM, 0.25 µM [³H]FPP, and the indicated compounds were incubated with this partially purified enzyme preparation. Data presented in Table 2 are the average of 2-5 determinations. Single capital letter amino acid abbreviations are used above.

The inhibition data indicates that Cys-Xaa¹-Xaa²-Xaa³-NRR¹ tetrapeptides of the invention bind to farnesyl-protein transferase. To further verify that Cys-Xaa¹Xaa²-Xaa³-NRR¹ were interacting with the active site of farnesyl-protein transfer, CVLS-NH₂ (SEQ ID NO: 1) was evaluated for its mechanism of inhibition using homogenous, pure farnesyl-protein transferase. CVLS-NH₂ (SEQ ID NO: 1) was observed to act as a competitive inhibitor with respect to Ras-CVLS protein substrate. CVLS-NH₂ (SEQ ID NO: 1) was not a competitive inhibitor with respect to farnesyl diphosphate. Thus, CVLS-NH₂ (SEQ ID NO: 1) binds to the protein substrate binding site of farnesyl-protein transferase, but CVLS-NH₂ (SEQ ID NO: 1) and tetrapeptides of the invention Cys-Xaa¹-Xaa²-Xaa³- NRR¹ do not serve as effective substrates in the reaction.

The compounds of this invention inhibit farnesyl-protein transferase and the farnesylation of the oncogene protein Ras. These compounds are useful as pharmaceutical agents for mammals, especially for humans. These compounds may be administered to patients for use in the treatment of cancer. Examples of the type of cancer which may be treated with the compounds of this invention include, but are not limited to, colorectal carcinoma, exocrine pancreatic carcinoma, and myeloid leukemias.

The compounds of this invention may be administered to mammals, preferably humans, either alone or, preferably, in combination with pharmaceutically-acceptable carriers or diluents, optionally with known adjuvants, such as alum, in a pharmaceutical composition, according to standard pharmaceutical practice. The compounds can be administered orally or parenterally, including intravenous, intramuscular, intraperitoneal, subsutaneous and topical administration.

For oral use of a chemotherapeutic compound according to this invention, the selected compounds may be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents include lactose and dried corn starch. When aqueous suspensions are required for oral use, the active ingredient is combined with emulsifying and suspending agents. If desired, certain sweetening and/or flavoring agents may be added. For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled in order to render the preparation isotonic.

The present invention also encompasses a pharmaceutical composition useful in the treatment of cancer, comprising the administration of a therapeutically effective amount of the compounds of this invention, with or without pharmaceutically acceptable carriers or diluents. Suitable compositions of this invention include aqueous solutions comprising compounds of this invention and pharmacologically acceptable carriers, e.g. saline, at a pH level e.g. 7.4. The solutions may be introduced into a patient's intramuscular blood-stream by local bolus injection.

When a compound according to this invention is administered into a human subject, the daily dosage will normally be determined by the prescribing physician with the dosage generally varying according to the age, weight, and response of the individual patient, as well as the severity of the patient's symptoms.

In one exemplary application, a suitable amount of compound is administered to a human patient undergoing treatment for cancer. Administration occurs in an amount between about .1 mg/kg of body weight to about 20 mg/kg of body weight of a mammal per day, preferably of between .5 mg/kg of body weight to about 10 mg/kg of body weight of a mammal per day.

### EXAMPLES

Examples provided are intended to assist in a further understanding of the invention. Particular materials employed, species and conditions are intended to be further illustrative of the invention and not limitative of the reasonable scope thereof.

### EXAMPLE 1

### Preparation of CVLS-NH₂(amide) (SEQ ID NO: 1)

Starting with 0.5 mmoles of p-methylbenzhydrylamine resin (p-MBHA) (which is commercially available) the following amino acids were introducted using an automated Applied Biosystems peptide synthesizer: t-boc-O-benzyl-L-serine, t-Boc-L-leucine, t-Boc-L-valine, t-Boc-S-4-methylbenzyl-L-cysteine. Couplings were mediated by dicyclohexylcarbodiimide to introduce each amino acid in its symmetrical anhydride form. Deblocking of the t-Boc group (tert-butyloxycarbonyl) was achieved with TFA (trifluoroacetic acid). At the completion of the assembly of the the protected peptide intermediate, t-Boc-S-4-methylbenzyl-L-cysteinyl-L-valyl-L-leucyl-0-benzyl-L-seryl-pMBHA resin, the t-Boc protecting group was removed with TFA and the resin bound peptide dried. The peptide was cleaved from the resin using liquid HF (hydrogen fluoride) in the presence of anisole as a scavenger at 0^{o}C 1 h. Following evaporation, the residual mixture was ether washed, filtered and the peptide extracted away from the resin with H₂O. The aqueous solution of crude product was freeze-dried. Purification of the peptide, Cys-Val-Leu-Ser-NH₂, (CVLS-NH₂) (SEQ ID NO: 1) was performed by reverse phase liquid chromatography on a C-18 silica based support. The gradient elution of the desired product was achieved using 0.1% aqueous TFA and 0.1% TFA in acetonitrile as buffers for elution. The purified product fractions were pooled and freeze-dried. Identity and homogeneity were established by amino acid composition, analytical HPLC and mass spectroscopy.

### EXAMPLE 2

### Preparation of CVFM-NH₂(amide) (SEQ ID NO: 2)

Starting with 0.5 mmoles of p-methyl benzhydrylamine resin (p-MBHA) (which is commercially available) the following amino acids were introduced using an automated Applied Biosystems peptide synthesizer: t-Boc-L-methionine, t-Boc-L-phenylalanine, t-Boc-L-valine, t-Boc-S-4-methylbenzyl-L-cysteine. Couplings were mediated by dicyclohexyl-carbodiimide to introduce each amino acid in its symmetrical anhydride form. Deblocking of the t-boc group (tert-butyloxycarbonyl) was achieved with TFA (trifluoroacetic acid). At the completion of the assembly of the protected peptide intermediate, t-Boc-S-4-methylbenzyl-L-cysteinyl-L-valyl-L-phenylalan yl-L-methionyl-p-MBHA resin, the t-Boc protecting group was removed with TFA and the resin bound peptide dried. The peptide was cleaved from the resin using liquid HF (hydrogen fluoride) in the presence of anisole as a scavenger at 0°C for 1 hour. Following evaporation, the residual mixture was ether washed, filtered and the peptide extracted away from the resin with H₂O. The aqueous solution of crude product was freeze-dried. Purification of the peptide, Cys-Val-Phe-Met-NH₂, (CVFM-NH₂) (SEQ ID NO: 2) was performed by reverse phase liquid chromatography on a C-18 silica based support. The gradient elution of the desired product was achieved using 0.1% aqueous TFA and 0.1% TFA in acetonitrile as buffers for elution. The purified product fractions were pooled and freeze-dried. Identity and homogeneity were established by amino acid composition, analytical HPLC and mass spectroscopy.

SEQUENCE LISTING

(1) GENERAL INFORMATION:

(i) APPLICANT: Gibbs, Jackson B
Garsky, Victor M
Rands, Elaine
Pompliano, David L

(ii) TITLE OF INVENTION: Non-Substrate Inhibitors of Farnesyl
Protein Transferase
(iii) NUMBER OF SEQUENCES: 2

(iv) CORRESPONDENCE ADDRESS:
(A) ADDRESSEE: Merck and Co., Inc.
(B) STREET: P.O. Box 2000, 126 E. Lincoln Avenue
(C) CITY: Rahway
(D) STATE: New Jersey
(E) COUNTRY: United States
(F) ZIP: 07065-0900

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: US 746,389
(B) FILING DATE: 16-AUG-1991
(C) CLASSIFICATION: original

(viii) ATTORNEY/AGENT INFORMATION:
(A) NAME: Daniel, Mark R
(B) REGISTRATION NUMBER: 31,913
(C) REFERENCE/DOCKET NUMBER: 18375

(ix) TELECOMMUNICATION INFORMATION:
(A) TELEPHONE: (908) 594-6609
(B) TELEFAX: (908) 594-4720

(2) INFORMATION FOR SEQ ID NO:1:

(i) SEQUENCE CHARACTERISTICS:
(A) LENGTH: 4 amino acids
(B) TYPE: amino acid
(C) STRANDEDNESS: unknown
(D) TOPOLOGY: linear

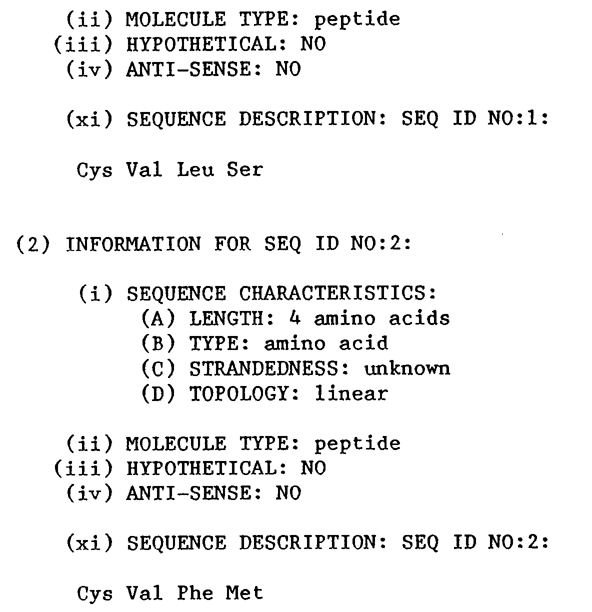

## Claims

1. A compound which inhibits farnesyl - protein transferase and prevents transformation of normal cells into cancer cells by acting as a non-substrate inhibitor, which compound comprises the amino acid sequence:
Cys - Xaa¹ - Xaa² - Xaa³ - NRR¹
wherein:
Cys is the amino acid Cysteine;
Xaa¹ is any amino acid in the natural L-isomer form;
Xaa² is any amino acid in the natural L-isomer form; and
Xaa³-NRR¹ is an amide of any amino acid in the natural L-isomer form, wherein R and R¹ are independently selected from hydrogen, C₁ - C₁₂ alkyl, aralkyl, or unsubstituted or substituted aryl; or the pharmaceutically acceptable salts thereof.

2. A compound according to Claim 1 which is CVLS-NH₂ (SEQ ID NO: 1).

3. A compound according to Claim 1 which is CVFM-NH₂ (SEQ ID NO: 2).

4. A chemotherapeutic composition comprising a pharmaceutical carrier, and dispersed therein, a therapeutically effective amount of a compound according to Claims 1 through 3.

5. The use of a compound as claimed in claim 1 for the manufacture of a medicament for inhibiting farnesyl-protein transferase and plasma membrane Ras protein localization, and preventing transformation of normal cells into cancer cells by acting as a non-substrate inhibitor.

6. The use of a compound as claimed in claim 1 for the manufacture of a medicament for the treatment of cancer.
